# EUROPEAN PATENT APPLICATION

(11) **EP 2 468 273 A2**
(43) Date of publication of application: **27.06.2012**
(21) Application number: 11193305.7
(22) Date of filing: 15.06.2006
(51) Int. Cl.: A61K 31/22, A61K 31/225, A61K 31/202, A61K 31/685, A61K 31/683, A61K 31/33, A61P 25/16, A61P 25/28, A61P 25/08, A61P 25/18, A61P 9/00, A61P 9/10, A61P 43/00, A61K 31/205, A61K 31/23, A61K 45/06

(54) **Method to reduce oxidative damage and improve mitochondrial efficiency**

(30) Priority: 20.06.2005 US 69232805 P
(62) Divisional of application: 06773269.3
(71) Applicant: Accera, Inc., Broomfield, Colorado 80021 (US)
(72) Inventor: Henderson, Samuel, Golden, Colorado 80403 (US)
(74) Representative: Clegg, Richard Ian

(57) **Abstract**

Methods for the reduction of mitochondrial oxidative damage and improved mitochondrial efficiency in an animal by administration of medium chain triglycerides or prodrug of medium chain triglycerides to the animal are provided.

## Description

### FIELD OF THE INVENTION

The present invention relates to the modulation of mitochondrial function in an animal. In particular, the present invention relates to the reduction of mitochondrial oxidative damage and improved mitochondrial efficiency by administration of medium chain triglycerides or prodrug of medium chain triglycerides to said animal.

### BACKGROUND OF THE INVENTION

Reactive oxygen species (ROS) generated from oxidative phosphorylation, which takes place in the mitochondria, can damage all classes of cellular components; lipids, proteins and nucleic acids. Damaged cellular components inhibit the normal function of the cell and are associated with numerous pathological conditions.

Unsaturated lipids are a major component of cellular membranes and are particularly vulnerable to oxidative damage. ROS species act on unsaturated lipids to yield reactive unsaturated aldehydes. These aldehydes can react with other cellular components, such as membrane bound or associated proteins and nucleic acids, thereby crosslinking them to the lipid. These reactions can greatly inhibit the function of membrane proteins and disturb membrane fluidity. In particular cross linked ETC complexes within mitochondrial membranes will inhibit mitochondrial function. Oxidized lipids are frequently identified by presence of lipid peroxides.

Proteins are also vulnerable to oxidation damage. In particular proteins containing sulfhydryl groups and iron-sulfur clusters are vulnerable to attack by ROS. For example, ROS attack on mitochondrial aconitase will cause release of the iron from the protein and inactivation of the enzyme. The released iron will also become available to generate hydroxyl radicals from superoxide and hydrogen peroxide. In addition, ROS attack on proteins can lead to crosslinking with lipids, nucleic acids and other proteins, thereby inhibiting a variety of cellular processes. Oxidized proteins are frequently identified by presence of protein carbonyls.

Nucleic acids are also vulnerable to ROS resulting in many forms of oxidized bases and DNA adducts. Such damaged bases can lead to decreased expression of damaged genes as well as mutations. Oxidized nucleic acids are frequently identified by presence of oxidized bases, such as 8-oxo-guanine.

Given the fundamental nature of mitochondrial function and protection from oxidative damage, there exists a need to improve electron transport efficiency and lower oxidative damage. In particular there is a need to reduce generation of reactive oxygen species with safe and effective treatment. It is the novel insight of the inventor that ingestion of medium chain triglycerides will meet this need.

### SUMMARY OF THE INVENTION

The present invention provides a method of modulating mitochondrial function in a mammal, comprising administering to the mammal an effective amount of an agent which induces development of ketosis in the mammal, whereby mitochondrial function is modulated. Administration may be oral or intravenous. Typically, the agent is administered in an effective amount. In some embodiments, agent is medium chain triglycerides. The medium chain triglycerides may be emulsified, and may be coadministered with L-carnitine or a derivative of L-carnitine.

In other embodiments, the agent is free fatty acids, such as those derived from medium chain triglycerides, or a medium chain triglyceride prodrug to a patient in need thereof

In one embodiment, the agent comprises a medium chain triglyceride and a sugar, including monosaccharides, disaccharides, polysaccharides, and mixtures thereof

In one embodiment, the agent comprises a medium chain triglyceride and a TCA cycle intermediate or a metabolic precursor of a TCA intermediate.

In one embodiment, the agent comprises a medium chain triglyceride and a ketone body or metabolic precursor of a ketone body, including β-hydroxybutyrate, acteoacetate, metabolic precursors of β-hydroxybutyrate or acteoacetate, a physiologically acceptable salt or ester of a polymer or oligomers, and mixtures thereof

In one embodiment, the agent comprises medium chain triglyceride and a metabolic adjuvant, such as a vitamin, a mineral, an antioxidant, an energy-enhancing compound, and mixtures thereof, Coenzyme CoQ-10, creatine, L-carnitine, n-acetyl-carnitine, L-canitine derivatives, and mixtures thereof

The present invention also provides therapeutic agents which are derivatives of medium chain triglycerides, and can include ketone body precursors and essential fatty acids esterified to a the glycerol backbone.

In one embodiment, the agent comprises medium chain triglyceride and a therapeutic agent selected from the group consisting of acetylcholinesterase inhibitors, acetylcholine synthesis modulators, acetylcholine storage modulators, acetylcholine release modulators, anti-inflammatory agents, estrogen or estrogen derivatives, insulin sensitizing agents, β-amyloid plaque removal agents (including vaccines), inhibitors of β-amyloid plaque formation, γ-secretase modulators, pyruvate dehydrogenase complex modulators, α-ketoglutarate dehydrogenase complex modulators, neurotrophic growth factors (e.g., BDNF), ceramides or ceramide analogs, and/or NMDA glutamate receptor antagonists.

In one embodiment, the agent comprises medium chain triglyceride and at least one therapeutic agent which induces utilization of fatty acids, including a PPAR-gamma agonist such as aspirin, ibuprofen, ketoprofen, and naproxen, and thiazolidinedione drugs, a statin drug such as Liptor or Zocor, fibrate drugs such as Bezafibrate, ciprofibrate, fenofibrate or Gemfibrozil, and other compounds that increase metabolism of fatty acids, such ascaffeine, and ephedra.

In one embodiment, the agent comprises an agent which induces utilization of fatty acids, including a PPAR-gamma agonist, a statin drug, and a fibrate drug.

In one embodiment, the agent comprises β-hydroxybutyrate, acteoacetate, metabolic precursors of β-hydroxybutyrate or acteoacetate, mixtures of the foregoing, and one selected from the group consisting of a PPAR-gamma agonist, a statin drug, and a fibrate drug.

In one embodiment, the agent comprises medium chain triglyceride and a triglyceride containing one or more essential fatty acids (EFAs) or precursors to EFAs, selecting from the group consisting of 18:2 n-6 (linoleic), 18:3 n-6, 20:3 n-6, 20:4 n-6 (arachidonic), 24:4 n-6, 24:5 n-6, 22:5 n-6, 18:3 n-3 (alpha-linolenic), 18:4 n-3, 20:4 n-3, 20:5 n-3 (eicosapetaenoic), 22:5 n-3, 2A:5 n-3, 24:6 n-3, 22:6 n-3 (docosahexanoic).

### BRIEF DESCRIPTION OF THE FIGURES

Figure 1 shows a schematic of oxidative phosphorylation

Figure 2 shows the melting point of various saturated fatty acids.

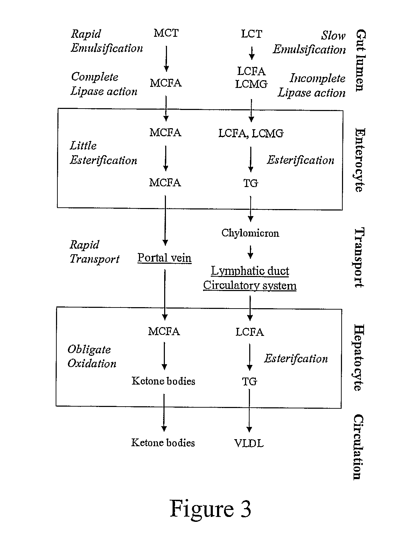

Figure 3 shows MCTs are metabolized differently from LCTs (Long Chain Triglycerides). MCTs are more rapidly emulsifed in the gut lumen, where pancreatic lipases hydrolyze them extensively to MCFA. In enterocytes, MCFA are poor substrates for esterification, and instead are exported directly into the portal vein. In enterocytes, LCFA are esterified back to LCT and packaged into chylomicrons which travel via lymph system before entering circulation. In the liver, MCFAs enter the mitochondria without use of carnitine and are undergo obligate oxidation. In the fed state LCFA entering the liver will be esterifed to glycerol and packaged into VLDL. **MCT-** medium chain triglyceride, **LCT** - long chain triglyceride, **MCFA** - medium chain fatty acid, **LCFA** - long chain fatty acid, **LCMG** - long chain monoglyceride, **TG**-triglyceride, **VLDL**-very low density lipoprotein.

### DETAILED DESCRIPTION OF THE INVENTION

It is the novel insight of this invention that agents that induce the development of ketosis, such as medium chain triglycerides (MCT) and their associated fatty acids, are useful as a treatment and preventative measure in patients who have a need for reducing oxidative damage and improving mitochondrial efficiency. As used herein, "patient" refers to any mammal, including humans, dogs, cats, rabbits, horses, cows, sheep, pigs, mink, or other mammals that may benefit from treatment of disease and conditions resulting from oxidative damage or impaired mitochondrial function. Accordingly, the present invention provides a method of modulating mitochondrial function in a mammal, the method comprising administering to the mammal an effective amount of an agent which induces development of ketosis in the mammal, whereby mitochondrial function is modulated. In Example 1, the inventor demonstrates the efficacy of tri-C8:0 medium chain triglycerides (MCTs) in reducing oxidative damage and improving mitochondrial efficiency in the dog model. In this example, MCTs induce elevated ketone body levels in the canine. Also, MCT treatments result in improved mitochondrial efficiency. Third, MCT treatments result in reduced signs of oxidative damage.

The physical differences between MCTs and long chain triglycerides (LCTs) results in three primary differences in the way these fats are metabolized. First, MCTs have a much more rapid rate and extent of digestion. Second, MCFAs derived from MCTs are rapidly absorbed by portal vein and are not transported by lymph system. Third, MCFAs undergo obligate oxidation in the mitochondria and are not subject to the regulation of LCFAs.

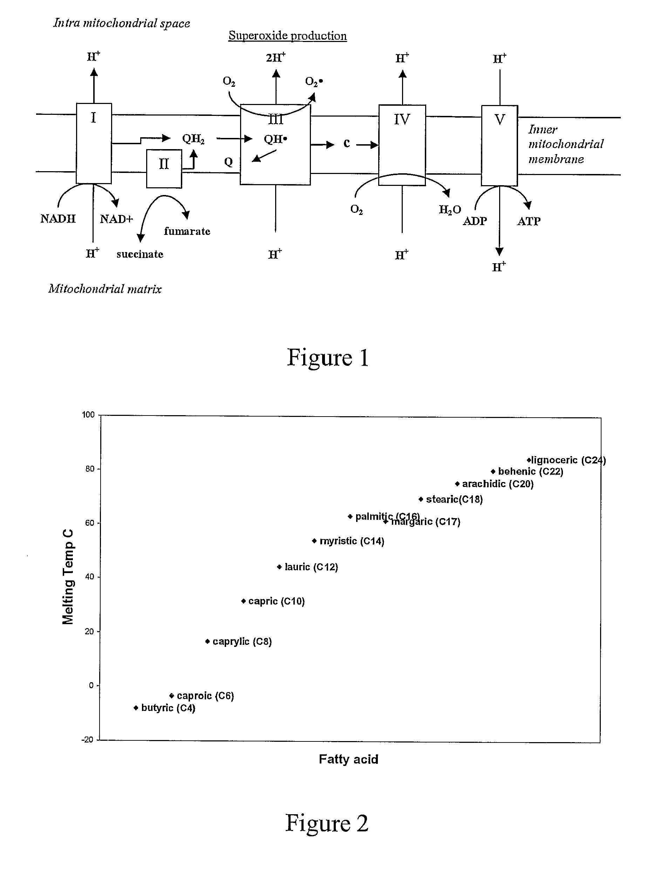

Due to the short chain length of the fatty acids in MCTs, they have much lower melting points than LCFAs. For example, the melting points of caprylic acid (C8:0) is 16.7°C and that of capric acid (C10:0) is 31.3°C, while the melting point of palmitic acid (C16:0) is much higher at 61.1°C (see Figure 2). As a result, both MCTs and MCFAs are liquid at room temperature.

MCFA are also smaller and more highly ionized than LCFA and, therefore, are much less hydrophobic and more soluble in biological fluids. For example, 9.7 grams of caproic acid (C6:0) will dissolve in 1 liter of water at 20°C, compared to only 0.007 grams of palmitic acid (C16:0). Therefore caproic acid is more than 1000 times more soluble in water as the much more common palmitic acid (See Table 1).

**Table 1. Solubility of fatty acids**

| Name | Carbon number | Solubility* |
|---|---|---|
| Acetic | 2 | To saturation |
| Butyric | 4 | To saturation |
| Caproic | 6 | 9.7 |
| Caprylic | 8 | 0.7 |
| Capric | 10 | 0.15 |
| Lauric | 12 | 0.055 |
| Myristic | 14 | 0.02 |
| Palmitic | 16 | 0.007 |
| Stearic | 18 | 0.003 |

| | | |
|---|---|---|
| **water at 20°C (in grams of acid per liter)* | | |

The lipid metabolizing enzymes in the gut are sensitive to the hydrophobicity of their subtrates. The increased solubility of MCFA and MCTs greatly influences their metabolism. As with LCTs, once ingested MCTs are acted upon by both lingual and gastric lipases and a small fraction of the released MCFAs are absorbed directly by the stomach. However, most MCT digestion occurs in the small bowel. Due to their increased solubility, MCTs have lower interfacial tension are more readily emulsified by lecithin and bile salts compared to LCTs. Hence, MCTs rapidly form fine emulsified particles, which are accessible to pancreatic lipase. Furthermore, MCFAs provide less allosteric inhibition on pancreatic lipase providing easy access to the fatty acid chains at positions 1 and 3 on the glycerol backbone. Medium chain 2-monoglycerides isomerizes much more rapidly to 1 or 3-monoglycerides than do long chain 2-monoglycerides, and this property greatly aids is the rapid and complete hydrolysis of MCTs. Therefore, most MCFAs are taken up by enterocytes and few medium chain monoglycerides are available for uptake.

Once LCFA are taken up by mucosa cells they are acted on by acyl-CoA synthetase and esterified to glycerol to generate LCTs which are packaged into chylomicrons and transported by lymph system. The acyl-CoA synthase has little activity on fatty acid chains of less than 12 carbons, hence MCFA are not good substrates for this enzyme and remain as Free Fatty Acids (FFA). Also, the ability of a fatty acid to be esterified to form TG is proportional to the ability of the fatty acid to bind to fatty-acid-binding-protein (FABP). LCFAs readily bind FABP while MCFAs do not. Thus MFCA are not re-esterified to MCTs, instead MCFAs enter the portal vein for transport to the liver. In this way MCTs are digested and resultant MCFAs transported to the liver much more rapidly than LCTs. In fact MCTs are digested with rates similar to glucose (for overview see (Babayan, V.K., Medium chain triglycerides and structured lipids, Lipids, 1987, 22:417-20)) The rapid digestion and transport of MCTs and MCFAs results in the liver being quickly perfused with MCFAs. Most of the MCFAs are retained in hepatocytes and only small amounts appear in the peripheral circulation.

In heptaocytes the major fate of MCFAs is oxidation. Hepatocytes contain long chain acyl-CoA synthetase enzymes in the endoplasmic reticulum and outer mitochondrial membrane, yet these are enzyme have little action on MCFAs. In addition hepatocytes contain FABP and will re-esterify LCFAs depending on the metabolic state of the organism As with enterocytes MCFAs do not efficiently bind FABP, instead MCFAs readily enter the mitochondria.

Entry of MCFA into the mitochondria is not dependent on carnitine transferases, such as CPT-I, and hence do not depend on the availability of carnitine. CPT-I represents and important control point in LCFA oxidation. Therefore MCFAs are immune to the regulation that is imposed on LCFA. This represents a critical difference between the metabolism of MCFAs

when compared to LCFAs. MCFAs undergo obligate oxidation in the liver regardless of the metabolic state of the organism. In contrast, the oxidation of LCFAs is tightly regulated by the physiologic state of the organism.

Within the hepatic mitochondrial matrix are medium chain acyl-CoA synthetases which activate MCFA for oxidation, and due to lack of regulation by CPT-I, MCFA are oxidized. The "uncontrolled" oxidation of MCFA leads to the production of large quantities of acetyl-CoA, and if sufficient quantities of MCTs are consumed, hepatocytes will generate ketone bodies. Since hepatocytes cannot utilize ketone bodies they are exported to the circulatory system for use by other tissues, in particular cerebral neurons. See Figure 3.

Without being bound by theory, it is believed that ketone bodies provide an energy source for extrahepatic cells, and that this energy source can reduce oxidative damage and improve mitochondrial efficiency as follows. Ketone bodies are produced in the liver by the oxidation ofMCTs, however, hepatocytes cannot metabolize ketone bodies because they lack the enzyme 3-oxoacid CoA transferase (acetoacetate:succinyl-CoA transferase), an enzyme necessary for the formation of acetoacetyl CoA. Therefore, ketone bodies are released by liver into the bloodstream for metabolism by extrahepatic tissues, such as neurons.

Ketone bodies are normally only produced under low glucose conditions such as during prolonged fasting or when very few carbohydrates are ingested. During these special conditions ketone bodies serve as a substitute for glucose. Ketone bodies are utilized by extrahepatic tissues through the conversion of β-hydroxybutyrate first to acetoacetate and then conversion of acetoacetate to acetoacetyl-CoA. The first step is catalyzed by β-hydroxybutyrate dehydrogenase, and the second by acetoacetate:succinyl-CoA transferase, also called 3-oxoacid CoA transferase. The conversion of β-hydroxybutyrate to acetoacetate generates NADH from NAD+, thereby increasing the reducing power of NADH/NAD+ couple.
Acetoacetate + Succinyl-CoA <------> Acetoacetyl-CoA + succinate

3-oxoacid CoA transferase is present is all tissues except the liver. This ensures that extrahepatic tissues have access to ketone bodies during prolonged starvation or when glucose levels are low. Acetoacetyl-CoA is converted into two molecules of acetyl-CoA by a thiolase :
Acetoacetyl-CoA + HS-CoA<------> 2 Acetyl-CoA

Acetyl-CoA may then enter the TCA cycle or be used in synthesis reactions.

In most organisms energy is obtained by the flow of electrons from reductants to oxidants in reduction-oxidation (redox) reactions. In eukaryotic mitochondria, electrons flow along an electron transport chain (ETC) and the released energy is used to pump protons across the inner mitochondrial membrane, generating a protonmotive force. This protonmotive force ultimately results in ATP synthesis. It is this chemiosmotic proton circuit, established by electron flow and proton pumping, that is the mechanism of oxidative phosphorylation.

Oxidative phosphorylation generates energy in the form of ATP and is also the major source of damaging reactive oxygen species (ROS) in living systems (Beckman, K.B. and Ames, B.N., The free radical theory of aging matures, Physiol Rev, 1998, 78:547-81). See Figure 1. During oxidative phosphorylation, superoxide, O2⁻•, may be generated. Superoxide is quickly dismutated to H₂O₂ spontaneously or by the action of the enzyme superoxide dismutase (SOD). However, in the presence of free transition metals (in particular iron and copper), O^{···}₂• and H₂O₂ will generate the extremely reactive hydroxylradical (•OH). Hydroxylradicals are believed to be the ROS species most responsible for damaging cellular components. Therefore, the rates of production of superoxide and H₂O₂ in many ways regulate the amount of cellular damage. ROS production in isolated mitochondria is largely dependent on the magnitude of the protonmotive force across the inner membrane. For example, if ADP becomes limiting (state IV respiration) protons will not flow through complex V and the potential across the inner mitochondrial membrane can not be dissipated and will increase, generating ROS (see Figure 1). Conversely, agents that increase respiration rates, such as uncouplers or ADP decrease ROS by dissipating the potential and lowering the protonmotive force.

Hence, it is the novel insight of the inventor that supplementation with agents which induce development of ketosis will improve mitochondrial efficiency and reduce ROS and will be of benefit to many conditions, such as Huntington's disease, Parkinson's disease, and epilepsy. Other related neurodegenerative diseases such as Wernicke-korsakoff disease and possibly schizophrenia will be benefited by high blood ketone levels, derived from MCT that reduce oxidative stress.

Reactive oxygen species generated from mitochondria have been implicated in numerous pathologoical conditions including the aging process itself (for overview see (Beckman, K.B. and Ames, B.N., The free radical theory of aging matures, Physiol Rev, 1998, 78:547-81). For example, oxidative damage and mitochondrial inefficiency have been implicated as central to the etiology of Alzheimer's disease. This has been described as a "mitochondrial spiral" in which poor functioning of the mitochondria leads to ROS. ROS in turn damages mitochondrial components leading to further decreases in mitochondrial function and a downward spiral of pathogenesis (Blass, J.P., Brain metabolism and brain disease: is metabolic deficiency the proximate cause of Alzheimer dementia?, J Neurosci Res, 2001, 66:851-6). Similar effects of ROS are well known to those skilled in the art for numerous medical conditions.

One agent which induces development of ketosis is a medium chain triglyceride. As used herein, a medium chain triglyceride is represented by the following formula: 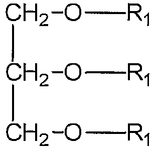 wherein R₁ is independently selected from the group consisting of a fatty acid residue esterified to a glycerol backbone having 5-12 carbons in the carbon backbone (C₅ to C₁₂ fatty acids), a saturated fatty acid residue esterified to a glycerol backbone having 5-12 carbons in the carbon backbone (C₅ to C₁₂ fatty acids), an unsaturated fatty acid residue esterified to a glycerol backbone having 5-12 carbons in the carbon backbone (C₅ to C₁₂ fatty acids), and derivatives of any of the foregoing. The MCTs of this invention may be prepared by any process known in the art, such as direct esterification, rearrangement, fractionation, transesterification, or the like. For example the lipids may be prepared by the rearrangement of a vegetable oil such as coconut oil. In general, MCTs are not commonly encountered in the human food chain. The most abundant natural sources are tropical plant oils such as coconut and babassu nut oil (see Table 2), and even these contain relatively low percentage of Capric (C10:0) and Caprylic (C8:0) fatty acids. Purified MCTs are typically prepared by hydrolysis of coconut oil, fractionation and purification of Medium Chain Fatty Acids (MFCA), and esterification to glycercol.

**Table 2. Composition of MCT containing oils**

| Fatty acid | Babassu | Coconut | Type of oil Cohune | Palm kernel | Tacum |
|---|---|---|---|---|---|
| Caproic(6:0) | 0.4 | 0.5 | 0.3 | 0.3 | 0.2 |
| Caprylic(8:0) | 5.3 | 8.0 | 8.7 | 3.9 | 2.9 |
| Capric(10:0) | 5.9 | 6.4 | 7.2 | 4.0 | 2.3 |
| Undecanoic(11:0) | - | - | 0.1 | - | - |
| Lauric(12:0) | 44.2 | 48.5 | 47.3 | 49.6 | 51.8 |
| Myristic(14:0) | 15.8 | 17.6 | 16.2 | 16.0 | 22.0 |
| Palmitic(16:0) | 8.6 | 8.4 | 7.7 | 8.0 | 6.8 |
| Stearic(18:0) | 2.9 | 2.5 | 3.2 | 2.4 | 2.3 |
| Oleic(18:1 n9) | 15.1 | 6.5 | 8.3 | 13.7 | 9.3 |
| Linoleic(18:2 n6) | 1.7 | 1.5 | 1.0 | 2.0 | 2.4 |
| Arachidic(20:0) | 0.1 | 0.1 | - | 0.1 | - |

| | | | | | |
|---|---|---|---|---|---|
| Adapted from (Babayan, V.K., Medium chain triglycerides and structured lipids, Lipids, 1987, 22:417-20) | | | | | |

In one embodiment, MCTs are MCTs wherein each R1 is a fatty acid containing a six-carbon backbone (tri-C8:0). Tri-C8:0 MCT are absorbed very rapidly by the gastrointestinal track in a number of animal model systems (Odle, J., New insights into the utilization of medium-chain triglycerides by the neonate: observations from a piglet model, J Nutr, 1997, 127:1061-7). The high rate of absorption results in rapid perfusion of the liver, and a potent ketogenic response. Additionally, utilization of MCT can be increased by emulsification. Emulsification of lipids increases the surface area for action by lipases, resulting in more rapid hydrolysis. Methods for emulsification of these triglycerides are well known to those skilled in the art.

The present invention provides methods of mo dulating mitochondrial function in a mammal. The term "to modulate mitochondrial function" means that the function of the mitochondria is altered when compared to not adding an agent. Modulation may occur on any level that affects function. A mitochondrial function may be direct or indirect, and measured directly or indirectly. In eukaryotic mitochondria, electrons flow along an electron transport chain (ETC) and the released energy is used to pump protons across the inner mitochondrial membrane. Electrons are fed into the electron transport chain from reduced substrates (e.g. glycerol phosphate, fatty acids, NADH or succinate) and pass down a gradient ofredox potential. Enzyme complexes, located within mitochondria membranes, such as NADH-Q oxidoreductase (Complex I) or succinate-Q oxidoreductase (Complex II)) pass electrons down the gradient ofredox potential to the lipid soluble carrier ubiquinone (Q) (See Figure 1). Reduced Q then transfers electrons through the Q-cytochrome c oxidoreductase enzyme complex (Complex III) to cytochrome c (a second water-soluble mobile carrier). Cytochrome c then donates electrons to the final acceptor, oxygen, in the cytochrome c oxidase complex (Complex IV). The energy derived from these oxidation reactions is used to establish an electrochemical gradient across the inner mitochondrial membrane. As electrons flow down the chemical gradient, Complex I, Complex III and Complex IV pump protons from the mitochondrial matrix into the intermembrane space, against their electrochemical gradient (Figure 1). This active plumping establishes a protonmotive force, consisting mainly of an electrical gradient (membrane potential) and a small chemical gradient (pH difference). This protonmotive force is used to drive protons back into the matrix through another enzyme complex, the mitochondrial ATP synthase (Complex V) resulting in ATP synthesis. It is this chemiosmotic proton circuit, established by electron flow and proton pumping, that is the mechanism of oxidative phosphorylation.

The rates of mitochondrial respiration can be controlled by several conditions, defined as states of respiratory control (see Table 3).

**Table 3 States of respiratroy control**

| State | Limiting conditions |
|---|---|
| I | Availability of ADP and subtrates |
| II | Availability of substrate only |
| III | Capacity of ETC itself |
| IV | Availability of ADP only |
| V | Availability of Oxygen only |

In the resting state mitochondria are typically in state IV and rates of respiration are controlled by availability ofADP. During intensive exercise the mitochondria may reach state III or state V, when the capacity of the ETC is maximized or oxygen can't be delivered fast enough to muscle tissue. In addition to being control points of mitochondrial function, States of respiration are useful measures of mitochondrial function. The States can be measured on purified mitochondria by adding or removing substrates and inhibitors. For example, addition ofuncouplers to purified mitochondria, uncouple electron flow from ATP production and is used to measure the maximum capacity of the ETC.

Mitochondrial function therefore includes, but is not limited to, State II respiration, State III respiration, State IV respiration, RCR, mitochondrial uncoupling protein activity, Complex 1-driven respiration (maximum electron transport capacity), and Complex II-driven respiration. In general, modulating mitochondrial function will result in reducing oxidative damage or improving mitochondrial efficiency. Methods of measuring mitochondrial function are known to those skilled in art and are referred to herein.

One method of measuring the function of mitochondria indirectly is to measure the oxidation oflipids and proteins. Since an ROS attack on proteins can lead to crosslinking with lipids, oxidized lipids may be identified by the presence of protein carbonyls and lipid peroxidation products. Oxidized proteins may also be identified by markers of protein oxidation such as peroxynitrite, or 3-nitrotyrosine.

The methods of the invention comprise administering to a mammal an agent which induces development of ketosis in the mammal. In some embodiments, the agent induces high blood ketone levels. As used herein, "high blood ketone levels" refers to levels of at least about 0.1 mM. In some embodiments, high blood ketone levels refers to levels of at least about 0.5 mM. In other embodiments, high blood ketone levels refers to levels of at least 1 mM. In general, the agent that induces development of ketosis is administered in an effective amount. In some embodiments, an effective amount is an amount sufficient to development of ketosis. In other embodiments, an effective amount is an amount sufficient to modulate mitochondrial function. In some embodiments, an effective amount is an amount sufficient to reduce oxidative damage or improve mitochondrial efficiency.

In the case where the goal is to treat or prevent a disease associated with reduced mitochondrial function or increased oxidative damage, an effective amount is an amount effective to either (1) reduce the symptoms of the disease sought to be treated or (2) induce a pharmacological change relevant to treating the disease sought to be treated An effective amount includes an amount effective to: increase impaired function; slow the progression of the disease; or increase the life expectancy of the affected patient.

In some embodiments the agent that induces development of ketosis is a free fatty acid. Free fatty acids may be administered in a single carbon chain length, or a mixture. In some cases the free fatty acid is derived from medium chain triglycerides. Because MCT are metabolized to produce medium chain fatty acids, which are oxidized, the administration of free fatty acids and/or ketone bodies have the same effect as the administration of MCT themselves.

In another embodiment, the invention comprises the coadministration of MCT, for example, emulsified tri-C8:0 MCT, and L-carnitine or a derivative of L-carnitine. Slight increases in MCFA oxidation have been noted when MCT are combined with L-carnitine (Odle, 1997). Thus in the present invention emulsified MCT are combined with L-carnitine at doses required to increase the utilization of said MCT. The dosage of L-carnitine and MCT will vary according to the condition of the host, method of delivery, and other factors known to those skilled in the art, and will be of sufficient quantity to raise blood ketone levels to a degree required to reduce oxidative damage and improve mitochondrial efficiency. Derivatives of L-carnitine which may be used in the present invention include but are not limited to decanoylcarnitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine.

Therapeutically effective amounts of the therapeutic agents can be any amount or dose sufficient to bring about the desired effect and depend, in part, on the severity and stage of the condition, the size and condition of the patient, as well as other factors readily known to those skilled in the art. The dosages can be given as a single dose, or as several doses, for example, divided over the course of several weeks.

In one embodiment, the agent is administered orally. In another embodiment, the agent is administered intravenously. Oral administration of MCT and preparations of intravenous MCT solutions are well known to those skilled in the art. In the present invention it is revealed that administration of MCTs increases ketone body levels, thereby modulating mitochondrial function, reducing oxidative damage and improving mitochondrial efficiency.

In another embodiment, the invention provides the subject compounds in the form of one or more prodrugs, which can be metabolically converted to the subject compounds by the recipient host. As used herein, a prodrug is a compound that exhibits pharmacological activity after undergoing a chemical transformation in the body. The said prodrugs will be administered in a dosage required to modulate mitochondrial function. A wide variety of prodrug formulations are known in the art. For example, prodrug bonds may be hydrolyzable, such as esters or anhydrides, or enzymatically biodegradable, such as amides.

This invention also provides a therapeutic and nutraceutical compositions capable of modulating mitochondrial function. In some embodiments, the compositions comprise medium chain triglycerides. The compositions may be administered conventionally (in tablets or capsules) as a dietary supplement, or be delivered as a food additive, food or beverage product, functional food or beverage, medical food, botanical drug, over-the-counter (OTC) drug, prescription drug, compounded drug, or any other category ofproduct for ingestion by human beings regulated by the Food and Drug Administration (FDA) which may be created, defmed, or re-named in the future. The compositions in their ingested embodiment may take any form: pill, capsule, soft gel, liquid extract, chewable, powdered shake, health drink, protein bar, or meal replacement. In one embodiment, the composition is provided in administratively convenient formulations of the compositions including dosage units incorporated into a variety of containers. In some embodiments, dosages of the MCT are administered in an effective amount, in order to improve the condition of mammals afflicted by a variety of disorders where reducing oxidative damage and improving mitochondrial efficiency would be beneficial. In one embodiment, an MCT dose will be in the range of 0.05 g/kg/day to 10 g/kg/day of MCT. In other embodiments, the dose will be in the range of 0.1 g/kg/day to 5 g/kg/day of MCT. In other embodiments, the dose will be in the range of 0.2 g/kg/day to 1 g/kg/day of MCT. Convenient unit dosage containers and/or formulations include tablets, capsules, lozenges, troches, hard candies, nutritional bars, nutritional drinks, metered sprays, creams, and suppositories, among others. The compositions may be combined with a pharmaceutically acceptable excipient such as gelatin, an oil, and/or other pharmaceutically active agent(s). For example, the compositions may be advantageously combined and/or used in combination with other therapeutic or prophylactic agents, different from the subject compounds. In many instances, administration in conjunction with the subject compositions enhances the efficacy of such agents. For example, the compounds may be advantageously used in conjunction with antioxidants, compounds that enhance the efficiency of glucose utilization, and mixtures thereof, (see e.g. Goodman et al. 1996).

In one embodiment MCTs are combined with triglycerides containing essential fatty acids (EFAs), selecting from the group consisting of 18:2 n-6 (linoleic), 18:3 n-6 , 20:3 n-6, 20:4 n-6 (arachidonic), 24:4 n-6, 24:5 n-6, 22:5 n-6, 18:3 n-3 (alpha-linolenic), 18:4 n-3, 20:4 n-3, 20:5 n-3 (eicosapetaenoic), 22:5 n-3, 24:5 n-3, 24:6 n-3, 22:6 n-3 (docosahexanoic). EFAs are incorporated into lipid membranes, such as mitochondrial membranes, where they may modulate mitochondrial function. For example, cardiolipin is a major phospholipids found in the inner mitochondrial membrane. The fatty acid chain associated with cardiolipin is an essential fatty acid. The EFA is normally (18:2(n-6)) however it may vary based on diet. Supplementation with triglycerides containing n-3 fatty acids can lead to the substitution of (18:2(n-6)) with (18:2(n-3)). Changing the composition of cardiolipin in this way will modulate the activity of enzymes involved in oxidative phosphorylation. It is the novel insight of the inventor that co-administration of MCTs and triglycerides containing EFAs will modulate mitochondrial function and result in reduction of oxidative stress and improvement in mitochondrial efficiency.

In one embodiment, the invention provides a formulation comprising a mixture of MCT and EFA containing long chain triglycerides (EFA:LCT). The nature of such formulations will depend on the duration and route of administration. Such formulations will be in the range of 0.05 g/kg/day to 10 g/kg/day of MCT and 0.01 g/kg/day to 5 g/kg/day of EFA:LCTs. In one embodiment, an MCT dose will be in the range of 0.05 g/kg/day to 10 g/kg/day of MCT. In other embodiments, the dose will be in the range of 0.1 g/kg/day to 5 g/kg/day of MCT. In other embodiments, the dose will be in the range of 0.2 g/kg/day to 1 g/kg/day of MCT. In some embodiments, EFA:LCTs will be in the range of 0.01 g/kg/day to 10 g/kg/day. In other embodiments, the EFA:LCT dose will be in the range of 0.01 g/kg/day to 1 g/kg/day. In still other embodiments, the EFA:LCT dose will be in the range of 0.02 g/kg/day to 0.75 g/kg/day Variations will necessarily occur depending on the formulation and/or host, for example.

One formulation comprises a range of 1-500 g of emulsified MCT combined with 1-2000 mg of EFA:LCTs. Another formulation comprises 50 g MCT (95% triC8:0) emulsified with 50 g of mono- and di-glycerides combined with 500 mg of EFA:LCTs. Such a formulation is well tolerated in healthy human subjects.

In one embodiment the human subject is intravenously infused with MCT and/or MCFA (medium chain fatty acids) directly, to a level required to modulate mitochondrial efficiency. Preparation of intravenous lipid solutions is well known to those skilled in the art.

In one embodiment, the invention provides a formulation comprising a mixture of MCT and carnitine. The nature of such formulations will depend on the duration and route of administration. Such formulations will be in the range of 0.05 g/kg/day to 10 g/kg/day of MCT and 0.05 mg/kg/day to 10 mg/kg/day of carnitine or its derivatives. In one embodiment, an MCT dose will be in the range of 0.05 g/kg/day to 10 g/kg/day of MCT. In other embodiments, the dose will be in the range of 0.1 g/kg/day to 5 g/kg/day of MCT. In other embodiments, the dose will be in the range of 0.2 g/kg/day to 1 g/kg/day of MCT. In some embodiments, a carnitine or carnitine derivative dose will be in the range of 0.01 g/kg/day to 10 g/kg/day. In other embodiments, the carnitine or carnitine derivative dose will be in the range of 0.01 g/kg/day to 1 g/kg/day. In still other embodiments, the canitine or carnitine derivative dose will be in the range of 0.02 g/kg/day to 0.75 g/kg/day. Variations will necessarily occur depending on the formulation and/or host, for example.

One formulation comprises a range of 1-500 g of emulsified MCT combined with 1-2000 mg of carnitine. Another formulation comprises 50 g MCT (95% triC8:0) emulsified with 50 g of mono- and di-glycerides combined with 500 mg of L-carnitine. Such a formulation is well tolerated in healthy human subjects.

In another embodiment, the invention provides the recipient with a therapeutic agent which enhances endogenous fatty acid metabolism by the recipient. The said therapeutic agent will be administered in a dosage required to modulate mitochondrial function in a patient in need thereof Ketone bodies are produced continuously by oxidation of fatty acids in tissues that are capable of such oxidation. The major organ for fatty acid oxidation is the liver. Under normal physiological conditions ketone bodies are rapidly utilized and cleared from the blood. Under some conditions, such as starvation or low carbohydrate diet, ketone bodies are produced in excess and accumulate in the blood stream Compounds that mimic the effect of increasing oxidation of fatty acids will raise ketone body concentration to a level to reduce oxidative damage and improve mitochondrial efficiency. Since the efficacy of such compounds derives from their ability to increase fatty acid utilization and raise blood ketone body concentration they are dependent on the embodiments of the present invention.

. Compounds that mimic the effect of increasing oxidation of fatty acids and will raise ketone body concentration include but are not limited to the ketone bodies, D-β-hydroxybutyrate and aceotoacetate, and metabolic precursors of these. The term metabolic precursor, as used herein, refers to compounds that comprise 1,3 butane diol, acetoacetyl or D-β-hydroxybutyrate moieties such as acetoacetyl-1-1,3-butane diol, acetoacetyl- D-β-hydroxybutyate, and acetoacetylglycerol. Esters of any such compounds with monohydric, dihydric or trihydric alcohols is also envisaged. Metabolic precursors also include polyesters of D-β-hydroxybutyrate, and acetoaoacetate esters of D-β-hydroxybutyrate. Polyesters ofD-β-hydroxybutyrate include oligomers of this polymer designed to be readily digestible and/or metabolized by humans or animals. These may be 2 to 100 repeats long, typically 2 to 20 repeats long, and most conveniently from 3 to 10 repeats long. Examples of poly D-β-hydroxybutyrate or terminally oxidized poly-D-β-hydroxybutyrate esters useable as ketone body precursors are given below: 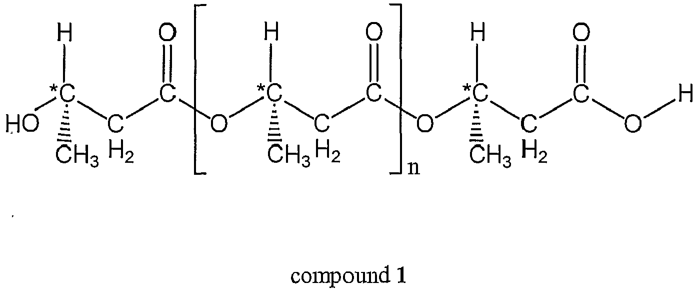 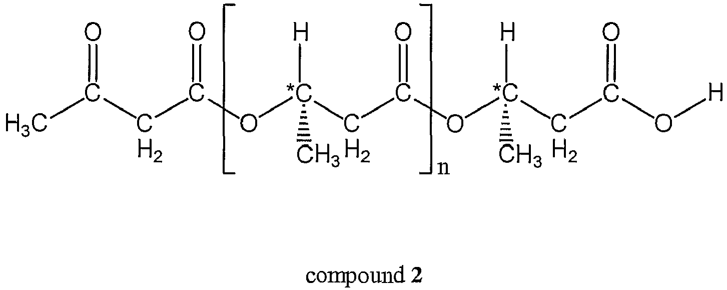 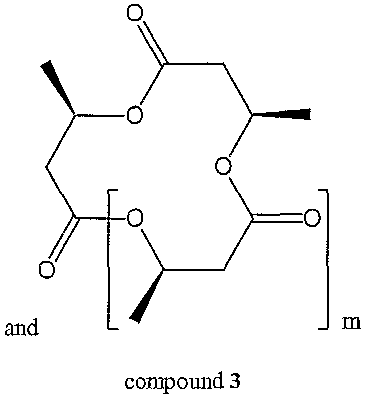 In each case n is selected such that the polymer or oligomer is readily metabolized on administration to a human or animal body to provide elevated ketone body levels in blood. Values ofn are integers of 0 to 1,000, in some cases 0 to 200, in some cases 1 to 50, in some cases 1 to 20, and in some cased from 3 to 5. In each case m is an integer of 1 or more, a complex thereof with one or more cations or a salt thereof for use in therapy or nutrition. Examples of cations and typical physiological salts are described herein, and additionally include sodium, potassium, magnesium, calcium, each balanced by a physiological counterion forming a salt complex, L-lysine, L-arginine, methyl glucamine and others known to those skilled in the art. The preparation and use of such metabolic precursors is detailed in Veech, WO 98/41201, and Veech, WO 00/15216, each of which is incorporated by reference herein in its entirety.

An additional metabolic precursor is a compound of the formula: 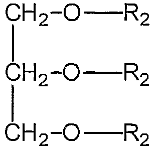 wherein R₂ is independently selected from the group consisting of R₁, β-hydroxybutyrate esterified to a glycerol backbone, acetoacetate esterified to the glycerol backbone, compound 1 esterified to a glycerol backbone, compound 2 esterified to a glycerol backbone, and compound 3 esterified to a glycerol backbone, with the proviso that at least one of R₂ is R₁. This compound will provide increased levels of ketone bodies due to the MCT character of the molecule, and the ketone body precursor character of the molecule. Accordingly, the present invention also provides a method of reducing oxidative damage and improving mitochondrial efficiency, comprising administering an effective amount of the foregoing compound to a patient in need thereof

Another embodiment consists of a dose of MCT combined with agents that increase the utilization of fats, MCT or ketone bodies. Examples of agents that increase utilization of fatty acids may be selected from a group comprising of, but not limited to, non-steroidal anti-inflammatory agents (NSAIDs), statin drugs (such as Lipitor® and Zocor®) and fibrates. Examples of NSAIDs include: aspirin, ibuprofen (Advil, Nuprin, and others), ketoprofen (Orudis KT, Actron), and naproxen (Aleve).

NSAIDs function, in part, as PPAR-gamma agonists. Increasing PPAR-gamma activity increases the expression of genes associated with fatty acid metabolism such as FATP (for review see (Gelman, L., et al., An update on the mechanisms of action of the peroxisome proliferator-activated receptors (PPARs) and their roles in inflammation and cancer, Cell Mol Life Sci, 1999, 55:932-43)). Accordingly, a combination of MCT and PPAR-gamma agonists will prove beneficial to reduce oxidative damage and improve mitochondrial efficiency. In one embodiment the PPAR-gamma agonist is an NSAID.

Statins are a class of drugs with pleiotropic effects, the best characterized being inhibition of the enzyme 3-hydroxy-3-methylglutaryl CoA reductase, a key rate step in cholesterol synthesis. Statins also have other physiologic affects such as vasodilatory, antithrombotic, antioxidant, anti-proliferative, anti-inflammatory and plaque stabilizing properties. Additionally, statins cause a reduction in circulating triglyceride rich lipoproteins by increasing the levels of lipoprotein lipase while also decreasing apolipoprotein C-III (an inhibitor of lipoprotein lipase) (Schoonjans, K., et al., 3-Hydroxy-3-methylglutaryl CoA reductase inhibitors reduce serum triglyceride levels through modulation of apolipoprotein C-III and lipoprotein lipase, FEBS Lett, 1999, 452:160-4). Accordingly, administration of statins results in increased fatty acid usage, which can act synergistically with MCT administration. One embodiment of this invention would be combination therapy consisting of statins and MCT.

Fibrates, such as Bezafibrate, ciprofibrate, fenofibrate and Gemfibrozil, are a class of lipid lowering drugs. They act as PPAR-alpha agonists and similar to statins they increase lipoprotein lipase, apoAI and apoAII trauscription and reduce levels of apoCIII (Staels, B., et al., Mechanism of action of fibrates on lipid and lipoprotein metabolism, Circulation, 1998, 98:2088-93). As such they have a major impact on levels of triglyceride rich lipoproteins in the plasma, presumably by increasing the use of fatty acids by peripheral tissues. Accordingly, the present invention discloses that fibrates alone or in combination with MCT would prove beneficial to patients in need or reduced oxidative damage or improved mitochondrial efficiency.

Caffeine and ephedra alkaloids are commonly used in over the counter dietary supplements. Ephedra alkaloids are commonly derived from plant sources such as ma-huang (*Ephedra sinica*). The combination of caffeine and ephedra stimulate the use of fat. Ephedra alkaloids are similar in structure to adrenaline and activate beta-adenergic receptors on cell surfaces. These adenergic receptors signal through cyclic AMP (cAMP) to increase the use of fatty acids. cAMP is normally degraded by phosphodiesterase activity. One of the functions of caffeine is to inhibit phosphodiesterase activity and thereby increase cAMP mediated signaling. Therefore caffeine potentiates the activity of the ephedra alkaloids. Accordingly, the present invention discloses that ephedra alkaloids alone can provide a treatment or prevention for conditions of reduced neuronal metabolism Additionally, it is disclosed that ephedra alkaloids in combination with caffeine can provide a treatment or prevention for conditions of reduced neuronal metabolism. Accordingly, it is disclosed that a combination of MCT with ephedra, or MCT with caffeine, or MCT, ephedra alkaloids and caffeine together can provide a method of reducing oxidative damage and improving mitochondrial efficiency.

Ketone bodies are used by neurons as a source of Acetyl-CoA. Acetyl-CoA is combined with oxaloacetate to form citrate in the Krebs' cycle, or citric acid cycle (TCA cycle). It is important for neurons to have a source ofAcetyl-CoA as well as TCA cycle intermediates to maintain efficient mitochondrial function. Yet, neurons lose TCA cycle intermediates to synthesis reactions, such as the formation of glutamate. Accordingly, the present invention discloses that a combination of ketone bodies with a source of TCA cycle intermediates will improve mitochondrial efficiency. TCA cycle intermediates are selected from a group consisting of citric acid, aconitic acid, isocitric acid, α-ketoglutaric acid, succinic acid, fumaric acid, malic acid, oxaloacetic acid, and mixtures thereof One embodiment of the invention is a combination of TCA cycle intermediates with MCT in a formulation to increase efficiency of the TCA.

Another source of TCA cycle intermediates are compounds that are converted to TCA cycle intermediates within the body (TCA intermediate precursors). Examples of such compounds are 2-keto-4-hydroxypropanol, 2,4-dihydroxybutanol, 2-keto-4-hydroxybutanol, 2,4-dihydroxybutyric acid, 2-keto-4-hydroxybutyric acid, aspartates as well as mono- and di-alkyl oxaloacetates, pyruvate and glucose-6-phosphate. Accordingly, the present invention discloses that a combination of TCA intermediate precursors with MCTs will be beneficial for reducing oxidative stress and improving mitochondrial efficiency. Also, the present invention discloses that MCT combined with TCA intermediate precursors will be beneficial for patients in need of reduced oxidative damage and improved mitochondrial efficiency.

The present invention further discloses that additional sources of TCA cycle intermediates and Acetyl-CoA can be advantageously combined with MCT therapy. Sources of TCA cycle intermediates and Acetyl-CoA include mono- and di- saccharides as well as triglycerides of various chain lengths and structures.

Further benefit can be derived from formulation of a pharmaceutical composition that includes metabolic adjuvants. Metabolic adjuvants include vitamins, minerals, antioxidants and other related compounds. Such compounds may be chosen from a list that includes but is not limited to; ascorbic acid, biotin, calcitriol, cobalamin, folic acid, niacin, pantothenic acid, pyridoxine, retinol, retinal (retinaldehyde), retinoic acid, riboflavin, thiamin, benfotiamine, a-tocopherol, phytylmenaquinone, multiprenylmenaquinone, calcium, magnesium, sodium, aluminum, zinc, potassium, chromium, vanadium, selenium, phosphorous, manganese, iron, fluorine, copper, cobalt, molybdenum, iodine. Accordingly a combination of ingredients chosen from: metabolic adjuvants, compounds that increase ketone body levels, and TCA cycle intermediates, will prove beneficial for reducing oxidative damage and improving mitochondrial efficiency.

Additional metabolic adjuvants include energy enhancing compounds, such as Coenzyme CoQ-10, creatine, L-carnitine, n-acetyl-camitine, L-carnitine derivatives, and mixtures thereof These compounds enhance energy production by a variety of means. Carnitine will increase the metabolism of fatty acids. CoQ10 serves as an electron carrier during electron transport within the mitochondria. Accordingly, addition of such compounds with MCT will increase metabolic efficiency especially in individuals who may be nutritionally deprived.

Further benefit can be derived from formulation of a pharmaceutical composition comprising MCT and other therapeutic agents which are used in the treatment of specific diseases, such as Alzheimer's disease, Parkinson's disease, Huntington's disease, or epilepsy. Such therapeutic agents includes acetylcholinesterase inhibitors, acetylcholine synthesis modulators, acetylcholine storage modulators, acetylcholine release modulators, anti-inflammatory agents, estrogen or estrogen derivatives, insulin sensitizing agents, β-amyloid plaque removal agents (including vaccines), inhibitors of β-amyloid plaque formation, γ-secretase modulators, pyruvate dehydrogenase complex modulators, neurotrophic growth factors (e.g., BDNF), ceramides or ceramide analogs, and/or NMDA glutamate receptor antagonists for overview of such treatments see (Bullock, R., New drugs for Alzheimer's disease and other dementias, Bs J Psychiatry, 2002, 180:135-9;Selkoe, D.J., Alzheimer's disease: genes, proteins, and therapy, Physiol Rev, 2001, 81:741-66)). While such treatments are still in the experimental stage it is the novel insight of the present invention that said treatments be combined with increased fatty acid/ketone body usage as described herein.

From the description above, a number of features of the invention for reducing oxidative stress and improving mitochondrial efficiency become evident:

(a) While prior art on preventing oxidative stress has been focused on antioxidants, the present invention provides a route for reducing oxidative stress based on the finding that feeding animals MCTs reduce signs of oxidative damage.

(b) While current treatments for oxidative damage have shown little efficacy and do not address the fundamental source of most reactive oxygen species, the mitochondria, ingestion of medium chain triglycerides as a nutritional supplement is a simple method to reduce oxidative stress and improve mitochondrial efficiency in a patient in need thereof

(c) Medium chain triglycerides can be infused intravenously into patients or administered orally.

Accordingly, the reader will see that the use of medium chain triglycerides (MCT) or fatty acids as a means to reduce oxidative stress and improve mitochondrial efficiency provides a means of treating people in need of such treatments. Although the description above contains many specificities, these should not be construed as limiting the scope of the invention but merely as providing illustrations for some of the presently disclosed embodiments of this invention. For example, supplementation with MCT may prove more effective when combined with insulin sensitizing agents such as vanadyl sulfate, chromium picolinate, and vitamin E. Such agents may function to increase glucose utilization in compromised neurons and work synergistically with MCTs. In another example MCTs can be combined with compounds that increase the rates of fatty acid utilization such as L-carnitine and its derivatives. Mixtures of such compounds may synergistically reduce oxidative stress and improve mitochondrial efficiency.

Thus the scope of the invention should be determined by the appended claims and their legal equivalents, rather than by the examples given.

### EXAMPLES

### Example 1

Reducing oxidative damage and improving mitochondrial efficiency. Two groups of aged beagle dogs were tested for the effects of feeding MCT on oxidative damage and mitochondrial efficiency. The first group was maintained on MCTs (2 g/kg/day) and the second group on placebo control over a 2-month period. Blood samples were taken over 5 time points to monitor levels of the ketone body, beta-hydroxybutyrate (BHB). After two months of treatment, the dogs were anaesthetized and brain biopsies were collected for mitochondrial analysis. The animals were then euthanized and brain tissue was harvested for further analysis of mitochondrial function and protein levels. The animals receiving MCTs had increased levels of serum BHB. They also had healthier mitochondria, as evidenced by better coupling of respiration with ATP formation. Finally, the mitochondria showed decreased signs of oxidative damage.

The eight subjects (8-11 years) were divided into two equal sized groups, matched for age and sex. The first group was administered 2 g/kg/day ofMCTs, which was added to their maintenance diet, and the other received only the maintenance diet. The test substance was manually added to the dog's normal chow and introduced over three days, such that one-third of the maximum dose was administered on the first day, two-thirds on the second day and the full dose on the third day. The subjects were maintained on the test substance over a two-month period. Blood samples for BHB analysis were taken on: Baseline (Day 0), Day 4, Day 14, Day 28, Biopsy/Euthanasia (Day 56 or 57).

On the day of biopsy, the treatment group was administered 2 g/kg ofMCTs and the control group was administered an isocalonic solution of sucrose by an oral-gastric tube. Approximately two hours after dosing, the animals were anaesthetized and biopsies were taken from the frontal and parietal lobes. Immediately after removing the biopsy tissue, the animals were euthanized with an injectable euthanizing agent.

Using the biopsied material, brain mitochondria were isolated and the following parameters were assayed in real-time:
1. State II respiration
2. State III respiration
3. State IV respiration
4. Mitochondrial uncoupling protein activity.
5. Complex I-driven respiration (Maximum electron transport capacity)
6. Complex II-driven respiration

**Biopsy procedure**. Approximately 30 minutes prior to surgery, a blood sample was taken for BHB analysis and then the animals were pre-medicated with acepromazine. Animals were induced using isoflurane (inhaled) via a facemask. The animal then were intubated and maintained in a deep plane of anaesthesia using isoflurane. Once the animal was anaesthetized, a small portion of skull was removed using a trephine. Ruptured blood vessels were cauterized to prevent blood from contaminating the tissue samples. Once the appropriate brain region was exposed, a small tissue punch was removed for the mitochondrial assays. The regions were identified based on cortical landmarks (ex: central sulcus).

After the tissue punches were collected, the animal was immediately euthanized using Euthanyl. Once the animal was deceased, as verified by absence of a heartbeat, CSF samples were collected from the central canal using a needle and syringe and the remainder of the skull was removed using an oscillating saw and rongeurs. Once the brain was fully exposed, the spinal cord and cranial nerves were gently severed from the brain. The brain then was removed.

Tissue punches from each animal were taken from the right hemisphere. Tissue harvested for 3^{rd} party analyses also were collected from the right hemisphere. The remaining tissue (the left hemisphere and parts of the right hemisphere) was immediately frozen at -80 °C.

Mitochondrial Assays. Mitochondria were isolated using differential centrifugation and released from synaptosomes using a nitrogen cell disrupter (1000 psi for 5 minutes).

The respiration assays were conducted using standard polarography in a sealed, thermostated, stirred chamber containing a Clark-type electrode, which measures oxygen within the chamber. The system was calibrated with oxygen-saturated water, to standardize the quantification of oxygen consumption. The assays were conducted at 37 °C.

Once the mitochondria were inserted into the Oxytherm, complex I driven respiration (state II respiration) was measured by adding pyruvate and malate (2.5 and 5 mM). Subsequently, 150 µM ADP was added to induce state III respiration, which measured the rate of active respiration. Oligomycin was added to inhibit ATP synthase and put the mitochondria into state IV respiration To assess UCP activity, free fatty acids were added. FCCP then was added to completely uncouple the mitochondria by depolarizing the membrane potential, which caused maximum electron transport activity and maximum oxygen consumption. Finally, rotenone was added to inhibit complex I electron transport and succinate was added to measure maximum complex II-driven electron transport and oxygen consumption (complex II-driven respiration). The respiratory control ratio (RCR) was tabulated by dividing state III respiration rates by state IV respiration rates.

The remaining mitochondria were stored in 10 % DMSO at -80 °C and assayed for UCP isoforms by western blot and quantification of oxidative damage in the mitochondrial, P 1 and cytosolic fractions.

Mitochondria from the biopsied tissue were frozen and analyzed for oxidative damage using markers ofperoxynitrite, lipid peroxidation and 3-nitrotyrosine.

Serum BHB levels were assessed at five different time-points: (1) prior to treatment onset (T0), (2) after 4 days of treatment (T1), (3) after 14 days of treatment (T2), (4) after 28 days of treatment (T3), and (5) on the biopsy date (T4). The levels were analyzed using a repeated-measures ANOVA with time-point (T0 vs. T1 vs. T2 vs. T3 vs. T4) as a within-subject variable and group (control vs. MCT) as a between-subject variable. The analysis revealed a significant interaction between group and BHB sampling time-point [F(4,24) = 4.670; p = 0.006]. BHB levels were significantly higher in the MCT group at T3 (day 28, p = 0.041) and T4 (biopsy date, p = 0.012). The analysis also revealed a marginally significant effect of group [F(1,5) = 5.838; p = 0.052].

The respiration rates that were tabulated on-site were analyzed using repeated-measures ANOVAs. For each measure, brain region (frontal vs. parietal) was the within-subject variable and group (control vs. MCT) was the between-subject variable.

State II Respiration. The analysis revealed a significant interaction between group and region [F(1,6) = 10.258; p = 0.019]. Post-hoc tests revealed that the treatment group showed significantly less oxygen consumption per minute per mg of tissue than the control group in the parietal cortex [F(1,6) =14.007; p = 0.010].

State III Respiration. The analysis revealed a significant effect of group [F(1,6) = 15.470; p = 0.008]. The treatment group showed more oxygen consumption per minute than the control group in both the parietal and frontal cortices.

State IV Respiration. The analysis revealed a marginally significant effect of group [F(1,6) = 4.976; p = 0.067]. The treatment group showed slightly more oxygen consumption per minute than the control group in both the parietal and frontal cortices.

Uncoupling Protein (UCP) Activity. The analysis revealed a marginally significant group effect [F(1,6) = 4.222; p = 0.086]. The animals receiving MCTs consumed more oxygen per minute than the control animals in both the parietal and frontal cortices.

Complex I-Driven Respiration. The analysis revealed a significant effect of group [F(1,6) = 27.325; p = 0.002]. The treatment group showed a larger maximum electron transport activity than the control group.

Complex II-Driven Respiration. The analysis revealed a significant interaction between region and group [F(1,5) = 6.862; p = 0.047]. The treatment group consumed more oxygen per minute than the control in the parietal cortex.
Markers of Oxidative Damage. The markers of oxidative damage were analyzed using a repeated-measures ANOVA with region (frontal vs. parietal) and fraction (mitochondrial vs. P 1 vs. cytosolic) as the within-subject variables and group (control vs. MCT) as the between-subject variable.

Lipid Oxidation. The analysis revealed a significant effect of fraction [F(2,10) = 14.721; p < 0.001]. The mitochondrial fraction had significantly less lipid oxidation than either the P1 fraction (p = 0.019) or the cytosolic fraction (p = 0.029). There were no additional significant or marginally significant effects on lipid oxidation.

Nitrotyrosine (3NT). The analysis revealed a marginally significant effect of region [F(1,2) =11.696; p = 0.076]. The parietal cortex had higher levels of 3NT than the frontal cortex.

Correlations Between BHB Levels and Mitochondrial Function. A bivariate correlational analysis was conducted to determine the relationship between serum BHB levels and the measures of mitochondrial function. We used two different measures of serum BHB levels: T4 levels, which were taken on the day of biopsy, and average treatment levels, which are based on 4 different sampling time-points.

Serum BHB levels on T4 (the day of biopsy) correlated with:
- State III respiration in the parietal cortex (r = 0.743; p = 0.035)
- Complex I-driven respiration in the parietal cortex (r = 0.799; p = 0.017)
- Protein oxidation in the mitochondrial fraction from the parietal cortex (r = -0.822; p = 0.023)
- UCP2 levels in the frontal cortex (r = -0.687; p = 0.060)

Average serum BHB levels correlated with:
- State III respiration in the parietal cortex (r = 0.837; p = 0.009)
- State IV respiration in the parietal cortex (r = 0.713; p = 0.047)
- UCP activity in the parietal cortex (r = 0.892; p = 0.003)
- Complex I-driven respiration in the parietal cortex (r = 0.859; p = 0.006)
- Protein oxidation in the mitochondrial fraction from the parietal cortex (r = -0.704; p = 0.077)

In general, the mitochondrial respiration rates demonstrate that mitochondria from the treatment animals were more bioenergetic. Specifically, the electron-transport chain (ETC), which generates the proton gradient, demonstrated an increase in the capacity to phosphorylate ADP to ATP, the energy source of all cells.

There were several indices of improved bioenergetics within the mitochondria. The RCR, which is the ratio of state III to state IV respiration, provides a direct index of coupling between respiration (electron-transport chain; ETC) and ATP production within the mitochondria. Typical RCRs range from 3-10, depending on the substrate used, isolation procedures used and the health of the mitochondria. In the present study, mitochondria from the control group had a lower RCR than treatment animals, which suggests that respiration in mitochondria from treatment animals is better coupled to ATP production. This change in RCR was attributable solely to a significant increase in state III respiration in the treatment group. The ETC ofmitochondria from treated animals had a significantly greater capacity to generate the proton gradient necessary to drive ATP production. In fact, state IV respiration was increased in the treatment group, most likely as a result of proton leakage across the inner membrane via uncoupling proteins. Evidence for this explanation comes from the fact that the addition of free fatty acids to activate UCPs demonstrated more UCP activity in the treatment group. The increased UCP activity has been linked to decreased ROS production.

The treatment also appeared to have a larger impact on complex I-driven respiration, when compared to complex II-driven respiration. These findings suggest that one of the earliest signs of mitochondrial damage in aged beagles is occurring upstream of complex II and III, either in complex I itself or in the citric acid cycle (TCA). This is supported by the increase in maximum complex I-driven respiration in the treatment group but no difference in the inhibition of complex I-driven respiration by rotenone and in complex II-driven respiration initiated by succinate. This also indicates that the complexes of the ETC upstream of complex III are intact and not affected by treatment.

The results also indicated differences between brain structures. Most notably, mitochondria from the parietal cortex showed greater functional integrity and were more responsive to MCTs. These findings likely reflect regional differences in the development of age-dependent neuropathology. Previous research has shown that the frontal cortex of dogs shows earlier susceptibility to deposition of amyloid-β (Aβ) than any other brain region. Dogs begin to develop Aβ, a key neuropathological feature of AD, in the prefrontal cortex around 8 years of age but don't begin to show significant accumulations in the parietal cortex until approximately 12 years of age. Similarly, aged dogs also have increased markers of oxidative stress in the prefrontal cortex. In the present study, the animals were between 8-11 years and, therefore, were likely to have had significantly more age-related pathology in the frontal lobe than in the parietal lobe. The fact that MCTs were more effective at improving mitochondrial function in the parietal cortex than the frontal cortex, therefore, likely is due to the subjects showing a greater degree of neuropathology in the frontal lobes.

The findings of the present invention demonstrate that MCTs can decrease oxidative damage, as measured by peroxynitrite (protein oxidation), lipid oxidation and 3-nitrotyrosine (3NT), possibly by increasing UCP activity.
UCPs are mitochondrial transporter proteins that allow protons to leak back across the inner mitochondrial membrane, thereby decreasing the inner membrane potential. Several isoforms of this protein exist and show tissue-specific distributions.

The present example demonstrates that MCTs improving mitochondrial function in aged beagle dogs. Collectively, these findings suggest that 2 g/kg of MCTs improves mitochondria function and reduces ROS thereby decreasing oxidative damage. The results also suggest that the effectiveness varies as a function of brain region in aged animals.

### Example 2

Protection from hypoxia-ischemia. Ischemic events are common in both stroke and heart disease. By reducing oxidative damage and improving mitochondrial efficiency MCTs will provide protection and treatment for ischemia and ischemia/reperfusion events. An established rat hypoxia-ischemia model will be used. 7-day-old rat pups will be divided into three groups: control, hypoxia-ischemia plus dextrose, and hypoxia-ischemia plus MCT treatment. In MCT treated pups, 0.5 g/kg of MCTs will be administered by or al gavage 2 to 4 hours before hypoxia. To induce ischemia the right common carotid artery of will be ligated, followed by 90 minute of hypoxia (8% 02 and 92% N2) at 37 °C. After hypoxia, MCTs and dextrose will be administered daily for an additional 2 days in the test groups. To evaluate protective effects on MCTs, brain weight, morphology, TUNEL assay, and DNA laddering will be evaluated. In addition, mitochondria will be isolated from each group and examined for State II respiration, State III respiration, State IV respiration, Mitochondrial uncoupling protein activity, Complex I-driven respiration (Maximum electron transport capacity), and Complex II-driven respiration.

MCTs are expected to abolish or reduce mortality during this mild hypoxic insult. MCTs are predicted to prevent brain weight loss caused by hypoxia. Additionally, MCTs are anticipated to reduce DNA fragmentation, and decrease TUNEL-positive cells, two markers of apoptosis. Cell fractions from the biopsied tissue will also be analyzed for oxidative damage using markers of peroxynitrite, lipid peroxidation and 3-nitrotyrosine. MCT treatment will reduce signs of oxidative damage in brain tissues. MCTs are anticipated to reduce brain injury, especially apoptotic cell death after hypoxia-ischemia, and reduce signs of oxidative damage caused by hypoxia-ischemia. MCTs are anticipated to improve mitochondria efficiency of test group.

### Example 3

Treatment of Parkinson's disease (PD). PD is a sporadic condition of uncertain etiology. However, several lines of evidence suggest that a defect in oxidative phosphorylation contributes to its pathogenesis. For instance, 1-methyl-4-phenyl-1,2,3,6-tetrahydropyridine (MPTP) is a neurotoxin that blocks complex I (NADH-ubiquinone oxidoreductase) of the mitochondrial electron transport chain and when animals are exposed to MPTP it causes a condition similar to PD.

8- to 10-week-old male C57BL mice will be divided into four groups: control, MPTP treated plus dextrose, and MPTP treated plus MCT. In dextrose treated pups, 0.5 g/kg of dextrose will be administered by oral gavage 2 to 4 hours before MPTP treatment. In MCT treated pups, 0.5 g/kg ofMCTs will be administered by oral gavage 2 to 4 hours before MPTP treatment. Eachmouse will then be randomly assigned to receive four intraperitoneal injections of either MPTP (18 mg/kg of free base in saline) or saline at 2-hour intervals. Mice will subsequently be tested for motor performance on a rotarod. The rotarod will consist of four rotating rods of 3 cm diameter in separated compartments. Seven days after MPTP or saline injections the mice will be first pretrained three times (1 hour apart) using an accelerating mode. After the training sessions, the time on the rodwill be recorded for successive rotational speeds, and the overall rod performance (ORP) for each mouse calculated. It is anticipated that MCT treatment will lessen the decline in motor performance induce by MPTP.

Seven days after the last MPTP injection, mice will be killed and their brain tissue processed for immunohistochemical studies to examine levels of oxidative stress and effects of MPTP treatment. Cell fractions from the biopsied tissue will be frozen and analyzed for oxidative damage using markers ofperoxynitrite, lipid peroxidation and 3-nitrotyrosine. It is anticipated that MCT treated group will show fewer signs of oxidative damage. In addition mitochondria will be isolated from each group and examined for mitochondrial efficiency. It is anticipated that the MCT treated group will show improved mitochondrial efficiency.

### Example 4

Treatment ofpolyglutamine (polyQ) diseases. Polyglutamine diseases are late-onset, neurodegenerative disorders that arise from the expansion of CAG repeats in the coding sequence of a variety of proteins. A growing number of inherited neurodegenerative disorders have been found to be caused by polyQ expansions. These include spinobulbar muscular atrophy (SBMA), Huntington's disease (HD), dentatorubralpallidolusian atrophy (DRPLA), and six spinocerebellar ataxias (SCA-1, 2, 3, 6, 7, 17). PolyQ containing proteins have been linked with inducting oxidative damage to nucleic acids through defective metabolism (Giuliano, P., et al., DNA damage induced by polyglutamine-expanded proteins, Hum Mol Genet, 2003, 12:2301-9). Hence polyQ disorders will benefit from reduced oxidative damage and improved mitochondrial efficiency as described in the present invention.

Mice carrying a mutant polyQ containing transgene are used to show that MCT treatment reduces polyglutamine pathogenesis *in vivo.* Transgenic will be divided into three groups: control, transgenic mice plus dextrose, and transgenic mice plus MCTs. In dextrose treated pups, 0.5 g/kg of dextrose will be administered by oral gavage daily for 3 months In MCT treated pups, 0.5 g/kg of MCTs will be administered by oral gavage daily for 3 months. Mice that carry a transgene expressing exon 1 of the Huntingin protein with a polyQ coding region develop progressive motor dysfunction, neuronal inclusions, and neuropathology typical of HD. At the end of the treatment, mice in each group are tested for motor function, using a rotating rod The rotarod will consist of four rotating rods of 3 cm diameter in separated compartments. Near the end of treatment the mice will be first pretrained three times (1 hour apart) using an accelerating mode. After the training sessions, the time on the rod will be recorded for successive rotational speeds, and the overall rod performance (ORP) for each mouse calculated It is anticipated that MCT treatment will lessen the decline in motor performance induced by polyQ proteins.

After completion of motor testing, the brains of the animals are to be examined for the presence and extent of neuronal inclusions and signs of oxidative damage. Mice fed MCTs are expected to perform for longer times on the motor rod, reflecting significant rescue of motor function. Additionally, MCT treated animals will show decreased amount of oxidative damage and improved mitochondrial efficiency. In particular, damage to mitochondrial DNA in the form of 8-hydroxy-2-deoxyguanosine (OH8dG) will be decreased in animals with polyQ containing proteins.
The following numbered paragraphs contain statements of broad combinations of the inventive technical features herein disclosed:
1. A method of modulating mitochondrial function in a mammal, comprising administering to the mammal an effective amount of an agent which induces development of ketosis in the mammal, whereby mitochondrial function is modulated.
2. The method paragraph 1, wherein said agent comprises medium chain triglycerides.
3. The method of paragraph 2, wherein modulating mitochondrial function comprises reducing oxidative damage or improving mitochondrial efficiency.
4. The method of paragraph 2, further comprising detecting the modulation of mitochondrial function.
5. The method of paragraph 4, wherein the detecting comprises detecting modulation of State I respiration, detecting modulation of State II respiration, detecting modulation of State III respiration, detecting modulation of State IV respiration, detecting modulation of RCR, detecting modulation of uncoupling protein activity, detecting lipid oxidation, and detecting protein oxidation.
6. The method paragraph 2, wherein said medium chain triglycerides are administered medium chain triglycerides are administered at a dose of about 0.1 g/kg/day to about 10 g/kg/day.
7. The method of paragraph 6, further comprising coadministering L-carnitine or a derivative of L-carnitine.
8. The method paragraph 7, wherein said L-carnitine or said derivative of L-carnitine is administered at a dose of about 0.5 mg/kg/day to about 10 mg/kg/day.
9. The method of paragraph 2, wherein said medium chain triglycerides are emulsified.
10. The method of paragraph 9, further comprising coadministering L-carnitine or a derivative of L-carnitine.
11. The method of paragraph 10, wherein said emulsified medium chain triglycerides and L-carnitine or a derivative of L-carnitine are administered in a formulation comprising 10-500 g emulsified medium chain triglycerides and 10-2000 mg L-carnitine or derivative of L-carnitine.
12. The method of paragraph 2, wherein the agent further comprises a ketone body or metabolic precursor of a ketone body.
13. The method of paragraph 12, wherein the ketone body is selected from a group consisting of β-hydroxybutyrate, acteoacetate, metabolic precursors of β-hydroxybutyrate or acteoacetate, and mixtures thereof.
14. The method of paragraph 13, wherein the metabolic precursor is a physiologically acceptable salt or ester of a polymer or oligomers wherein in each case the number of subunit repeats is selected such that the polymer or oligomers is readily metabolized on administration to a human or animal to provide elevated ketone body levels in the blood.
15. The method of paragraph 14, wherein the metabolic precursor is selected from the group consisting of: 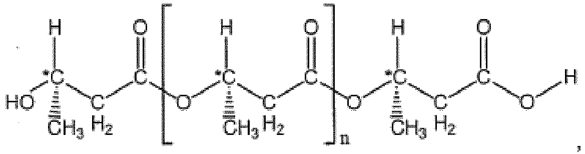 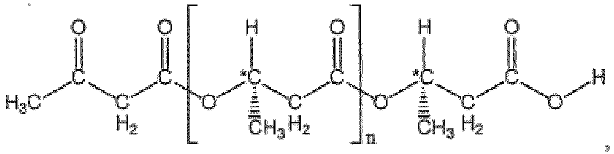 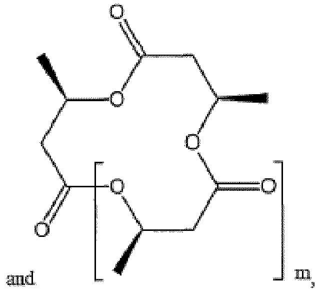 wherein n is an integer of 0 to 1,000, and m is an integer of 1 or more, a complex thereof with one or more cations or a salt thereof for use in therapy or nutrition.
16. The method of paragraph 2, wherein the agent further comprises a metabolic adjuvant.
17. The method of paragraph 16, wherein the adjuvant is selected from a group consisting of a vitamin, a mineral, an antioxidant, an energy-enhancing compound, and mixtures thereof
18. The method of paragraph 17, wherein the energy-enhancing compound is selected from a group consisting of Coenzyme CoQ-10, creatine, L-carnitine, n-acetyl-camitine, L-carnitine derivatives, and mixtures thereof
19. The method of paragraph 17, wherein the vitamin is selected from a group consisting of ascorbic acid, biotin, calcitriol, cobalamin, folic acid, niacin, pantothenic acid, pyridoxine, retinol, retinal (retinaldehyde), retinoic acid, riboflavin, thiamin, benfotiamine, a-tocopherol, phytylmenaquinone, multiprenylmenaquinone, pyridoxine derivatives, pantothenic acid, and mixtures thereof.
20. The method of paragraph 17, wherein the mineral is selected from a group consisting of calcium, magnesium, sodium, potassium, zinc, copper, aluminum, chromium, vanadium, selenium, phosphorous, manganese, iron, fluorine, cobalt, molybdenum, iodine and mixtures thereof
21. The method of paragraph 6 wherein the agent further comprises a triglyceride containing an essential fatty acid from a group consisting of 18:2 n-6 (linoleic), 18:3 n-6 , 20:3 n-6, 20:4 n-6 (arachidonic), 24:4 n-6, 24:5, n-6, 22:5 n-6, 18:3 n-3 (alpha-linolenic), 18:4 n-3, 20:4 n-3, 20:5 n-3 (eicosapetaenoic), 22:5 n-3, 24:5 n-3, 24:6 n-3, 22:6 n-3 (docosahexanoic).

## Claims

1. Medium chain triglycerides for use in a method of treatment of Parkinson's disease in a mammal wherein the method of treatment comprises administering a dose of medium chain triglycerides to the mammal of 0.1 g/kg/day to 5 g/kg/day and inducing ketosis in the mammal and reducing oxidative damage and/or improving mitochondrial efficiency in the mammal.

2. The medium chain triglycerides for use in a method of treatment according to claim 1 wherein the method of treatment comprises co-administration of L-carnitine or a derivative of L-carnitine to the mammal, wherein the derivative of L-carnitine is selected from decanoylcarnitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine.

3. The medium chain triglycerides for use in a method of treatment according to claim 2 wherein the method of treatment comprises administering a dose of said L-carnitine or said derivative of L-carnitine to the mammal of 0.05 mg/kg/day to 10 mg/kg/day, wherein the derivative of L-carnitine is selected from decanoylcarnitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine.

4. The medium chain triglycerides for use in a method of treatment according to any one of claims 1 to 3 wherein the medium chain triglycerides are emulsified.

5. The medium chain triglycerides for use in a method of treatment according to any one of claims 1 to 3 wherein the method of treatment comprises administering a formulation comprising 1-500 g emulsified medium chain triglycerides and 1-2000 mg L-carnitine.

6. The medium chain triglycerides for use in a method of treatment according to any one of claims 1 to 5 wherein the medium chain triglyceride is part of an agent, wherein the agent further comprises a ketone body.

7. The medium chain triglycerides for use in a method of treatment according to claim 6 wherein the ketone body is selected from a group consisting of β-hydroxybutyrate, acetoacetate, and mixtures thereof.

8. The medium chain triglycerides for use in a method of treatment according to claim 6 wherein the ketone body is a metabolic precursor of β-hydroxybutyrate or acetoacetate in the form of a physiologically acceptable salt or ester of a polymer or oligomers wherein in each case the number of subunit repeats is selected such that the polymer or oligomers is readily metabolized on administration to a human or animal to provide elevated ketone body levels in the blood, and is selected from the group consisting of: 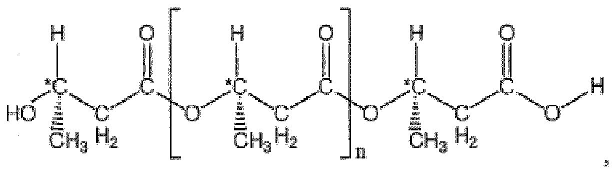 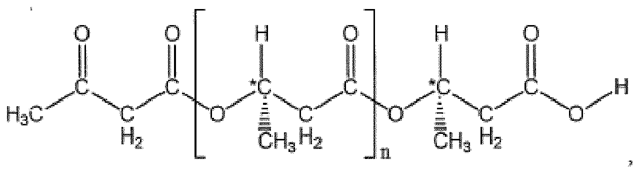 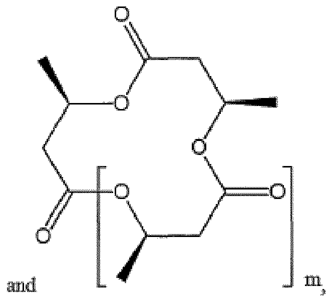 wherein n is an integer of 0 to 1000 and m is an integer of 1 or more, a complex thereof with one or more cations or a salt thereof for use in therapy or nutrition.

9. The medium chain triglycerides for use in a method of treatment according to any one of claims 1 to 5 wherein the medium chain triglyceride is part of an agent, wherein the agent further comprises a metabolic adjuvant selected from a group consisting of a vitamin, a mineral, an antioxidant, an energy-enhancing compound, and mixtures thereof wherein the energy-enhancing compound is selected from a group consisting of Coenzyme CoQ-10, creatine, L-carnitine, n-acetyl-carnitine, L-carnitine derivatives, and mixtures thereof and wherein the L-carnitine derivatives are selected from the group consisting of decanoylcarnitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine.

10. The medium chain triglycerides for use in a method of treatment according to claim 9 wherein the vitamin is selected from a group consisting of ascorbic acid, biotin, calcitrol, cobalamin, folic acid, niacin, pantothenic acid, pyridoxine, retinol, retinal (retinaldehyde), retinoic acid, riboflavin, thiamin, benfotiamine, a-tocopherol, phytylmenaquinone, multiprenylmenaquinone, pantothenic acid, and mixtures thereof.

11. The medium chain triglycerides for use in a method of treatment according to claim 9 wherein the mineral is selected from a group consisting of calcium, magnesium, sodium, potassium, zinc, copper, aluminum, chromium, vanadium, selenium, phosphorous, manganese, iron, fluorine, cobalt, molybdenum, iodine and mixtures thereof.

12. The medium chain triglycerides for use in a method of treatment according to any one of claims 1 to 5 wherein the medium chain triglyceride is part of an agent, wherein the agent further comprises a triglyceride containing an essential fatty acid from a group consisting of 18:2 n-6 (linoleic), 18:3 n-6, 20:3 n-6, 20:4 n-6 (arachidonic), 24:4 n-6, 24:5 n-6, 22:5 n-6, 18:3 n-3 (alpha-linoleic), 18:4 n-3, 20:4 n-3, 20:5 n-3 (eicosapetaenoic), 22:5 n-3, 24:5 n-3, 24:6 n-3, 22:6 n-3 (docosahexanoic).

13. The medium chain triglycerides for use in a method of treatment according to any one of claims 1 to 5 wherein the medium chain triglyceride is part of functional food.
